# EUROPEAN PATENT APPLICATION

(11) **EP 2 015 107 A2**
(43) Date of publication of application: **14.01.2009**
(21) Application number: 08270003.0
(22) Date of filing: 11.07.2008
(51) Int. Cl.: G01T 1/02, A61B 6/12, A61N 5/10

(54) **Trackable Implantable Sensor Devices, and Related Methods of Operation**

(30) Priority: 12.07.2007 US 949321 P
(71) Applicant: Sicel Technologies, Inc., Morrisville, NC 27560 (US)
(72) Inventor: Black, Robert D, Chapel Hill, NC 27517 (US); Mann, Gregory Glenwood, Raleigh, NC 27613 (US)
(74) Representative: Stainthorpe, Vanessa Juliet

(57) **Abstract**

An implantable biocompatible sensor unit includes a sensor body of a biocompatible material configured for *in vivo* placement proximate a tumor treatment site. The sensor body includes at least one sensor element are configured to provide data corresponding to treatment of the tumor treatment site. The sensor body further includes a transmitter coil and associated electronic components configured for wireless transmittal of the data to a spatially remote receiver. In addition, the sensor body includes a high-atomic weight member that is configured to be detectable by an imaging modality. The sensor unit is configured to be inductively powered to wirelessly transmit the data to the remote receiver while remaining implanted proximate the tumor treatment site. Related medical systems and methods of operation are also discussed.

## Description

### Cross Reference To Related Applications

This application claims priority to U.S. Provisional Application Serial No. 60/949,321, filed July 12, 2007, the disclosure of which is incorporated by reference herein in its entirety.

### Field of the Invention

The present invention relates to implantable sensor devices and related systems and methods.

### Background of the Invention

Radiation therapy is used to treat localized cancers or other conditions. Examples of radiation therapy treatments include conventional external beam radiation therapy, as well as three-dimensional conformal external beam radiation, intensity modulated radiation therapy (IMRT), a "gamma knife" that employs a highly focused gamma ray radiation obtained from crossing or collimating several radiation beams, stereotactic radiosurgery and brachytherapy.

The efficacy of the radiation treatment can depend on the total dose of radiation delivered to the target region. However, the amount of radiation effectively delivered to the target region (as well as the amount delivered to healthy tissue) can vary from a desired or planned amount. The variation can be particularly problematic when radiation therapy is used on deep tumors, when the therapy is delivered to tumors located close to healthy sensitive regions or organs, and/or when complex beam shapes are employed.

Typically, the radiation therapy is directed not only to the known tumor location, but also to healthy tissue proximate the tumor based on a treatment margin. Unfortunately, to compensate for the potential for imprecise radiation delivery, the planned treatment margin may be increased. As radiation can be detrimental to healthy tissue, a therapy goal should be to use smaller treatment margins while delivering radiation doses in the planned amounts and to the planned location. However, delivering external beam radiation doses in the desired dose amount to the actual tumor treatment site can be complicated as the tumor and/or markers used to locate and guide the radiation therapy may shift over time, either during or between radiation sessions.

For example, tumor motion can occur during the active delivery of the radiation due to normal biophysical actions. That is, in certain locations in the body, such as in the prostate, movement of target tissue may occur during radiation treatment, primarily attributable to the patient's breathing or filling and/or voiding of the bladder. Thus, dynamic changes in the position of the tumor during active radiation delivery can increase the potential of collateral damage to healthy or non-targeted tissue.

In the past, systems using positional markers have been proposed to determine spatial positioning information for targets from within a patient's body. Such localization may be used to direct or guide the radiation therapies. For example, ACCULOC® is an image-guided localization system that includes both hardware and software to provide localization based on implanted gold markers for an internal reference system. ACCULOC® employs gold spheres as bone markers in cranial and spinal applications, and cylindrical gold markers for soft tissue applications that can be inserted using a needle. These markers may be visible on standard port films as well as electronic portal imaging devices (EPID). Other procedures may also benefit from acquiring spatial knowledge during administration of a therapy to focus external energies to a desired targeted internal location, such as ultrasonic radiation treatments, microwave or RF ablation therapies, and localized ultrasonic or light activation of drugs.

Accordingly, there remains a need for improved or alternate techniques for providing localization data for target regions.

### Summary of the Invention

According to some embodiments of the present invention, an implantable biocompatible sensor unit includes a sensor body comprising a biocompatible material and having at least one sensor element configured *for in vivo* placement proximate a tumor treatment site. The sensor element is configured to provide data corresponding to treatment of the tumor treatment site. The sensor unit further includes a transmitter coil and associated electronic components within the sensor body configured for wireless transmittal of the data to a spatially remote receiver. In addition, the sensor unit includes a high-atomic weight member within the sensor body that is configured to be detectable by an imaging modality. The sensor unit is configured to be inductively powered to wirelessly transmit the data to the remote receiver while remaining implanted proximate the tumor treatment site.

According to other embodiments of the present invention, a medical system for patients undergoing treatment for cancer includes at least one wireless implanted sensor unit configured for *in vivo* placement proximate a tumor treatment site, and an external reader configured to inductively power the at least one sensor unit. The sensor unit includes a high-atomic weight member held in a biocompatible housing. The high-atomic weight member is configured to be visualized as a fiducial marker by an imaging modality. The sensor unit is configured to provide data including at least one sensed internal parameter, and is configured to be inductively powered to wirelessly transmit the data. The external reader is configured to receive the data from the at least one sensor unit, and the system is configured to dynamically monitor *selected in vivo* parameters associated with one or more of dose and/or processing of a therapy administered to the tumor treatment site based on the data from the at least one sensor unit.

According to further embodiments of the present invention, a method for treating a patient for cancer includes positioning at least one wireless sensor unit including at least one sensor element and a high-atomic weight member in the body of the patient proximate a tumor treatment site. A position of the sensor unit proximate to the tumor treatment site is determined using an imaging modality based on the high-atomic weight member. A radiation therapy is administered to the patient based on the determined position, and a signal is detected *in vivo* from the at least one sensor unit corresponding to the dose of the administered therapy proximate the tumor treatment site. Administration of the radiation therapy is also dynamically adjusted responsive to changes in the determined position.

Advantageously, the systems, methods, and devices of the present invention can act as positional markers and may monitor, in real time and/or dynamically, radiation dose, fluorescence, temperature, and/or specific indices associated with tumor physiology or other desired internal parameters to affirm therapeutic treatments. Embodiments of the present invention may be particularly suitable for oncology applications.

### Brief Description of the Drawings

**Figure 1** is a front view of an implantable sensor unit in a cylindrical housing according to embodiments of the present invention.
**Figure 2** is a side perspective view of a system including an implantable sensor unit that can define a positional marker and sense one or more internal parameters of interest according to some embodiments of the present invention.
**Figure 3** is a partial top view of internal components of an implantable sensor unit that is both a positional marker and a sensor for one or more internal parameters of interest according to some embodiments of the present invention.
**Figure 4A** is a partial top view of internal components of an implantable sensor unit that is both a positional marker and a sensor for one or more internal parameters of interest according to other embodiments of the present invention.
**Figure 4B** is a partial end view of the implantable sensor unit of **Figure 4A**.
**Figures 5** to **8** are partial top views of implantable sensor units that is both a positional marker and a sensor for one or more internal parameters of interest according to further embodiments of the present invention.
**Figure 9** is a schematic illustration of a medical imaging system that can detect an implantable sensor unit as a positional marker and a sensor configured to sense one or more internal parameters of interest according to some embodiments of the present invention.
**Figure 10** is a block diagram of a data processing system and/or computer modules according to embodiments of the present invention.
**Figure 11** is a block diagram of operations that may be used to carry out spatial positioning and radiation dose evaluations according to embodiments of the invention.
**Figure 12** is a schematic illustration of an imaging display including real-time gradient dose radiation data relative to spatial location of an internal tumor treatment site according to some embodiments of the present invention.

### Detailed Description of Embodiments of the Invention

The present invention now will be described more fully hereinafter with reference to the accompanying drawings, in which embodiments of the invention are shown. This invention may, however, be embodied in many different forms and should not be construed as limited to the embodiments set forth herein; rather, these embodiments are provided so that this disclosure will be thorough and complete, and will fully convey the scope of the invention to those skilled in the art. Like numbers refer to like elements throughout. In the drawings, like numbers refer to like elements throughout, and thickness, size and dimensions of some components, lines, or features may be exaggerated for clarity. The order of operations and/or steps illustrated in the figures or recited in the claims are not intended to be limited to the order presented unless stated otherwise.

Certain embodiments of the present invention are directed to operations that determine the *in situ* location of implanted sensor units that are adapted to detect, at sufficient intervals, one or more parameters, such as radiation received internally to a target tumor treatment site and/or to non-targeted healthy tissue or sensitive sites. The radiation doses may be delivered by an external beam therapy system. The target site may be any internal region undergoing analysis or therapy, and may be a site associated with tumors such as cancerous tissue. That is, the target site may be a localized cancerous tumor or a site where the tumor has been excised. As such, the excision site and/or tissue proximate thereto may be the target site. In certain embodiments, non-target sites may also be monitored for selected internal parameters or conditions. In particular embodiments, sensitive or non-target sites may be monitored to detect radiation dose received thereat, as desired. The radiation dose may be detected using additional implanted sensor units and/or disposable externally mounted radiation sensor patches. For additional discussion of suitable disposable (typically single-use) external radiation sensor patches, see co-pending U.S. Patent Application Serial No. 10/303,591, filed 11/25/02, the contents of which are hereby incorporated by reference as if recited in full herein.

Fiducial markers may be used to help align the patient and compensate for patient motion during external beam radiation therapy. Since many linear accelerators (LINACs) may not have the capability to image using diagnostic x-ray, it may be difficult to locate an implanted sensor unit, due to a lack of significant contrast with the surrounding soft tissue. Accordingly, in some embodiments of the present invention, a high-atomic weight material, such as gold, is added to the sensor unit (for example, in the form of a gold wire coil or clip). Some advantages of the use of a high-atomic weight member in an implantable sensor unit may include improved visualization on MV (mega voltage) images obtained during radiation therapy. For example, a sensor unit may be visible with relatively high contrast on diagnostic x-ray and/or CT images (at energies between about 80-120kVp). However, during radiation therapy, port films may be acquired using the treatment beam, which may usually be between about 6MV and about 18MV, and the sensor unit may not be as visible at these energies. Accordingly, when a high atomic weight material, such as gold, is added to the sensor unit, the sensor unit may be imaged with improved contrast during radiation therapy in relation to the surrounding tissue, and can be used to align the patient and/or compensate for patient motion through continuous tracking.

As such, the sensor units and methods of the present invention can be useful in many applications, such as, for example, pulmonary, gastrointestinal, neuroscience and pre-clinical research. Nonetheless, the present invention is believed to have particular importance and suitability *for in vivo* treatment of cancer.

In certain embodiments, the sensor units can be implanted relatively deep in the body of the subject and may remain in the body for a 1-8 week period or even longer in order to *provide in vivo* evaluation and monitoring of tumors prior to, during, and subsequent to an active treatment, and preferably over an entire treatment regime or period. As such, the internal *in situ* sensors of the present invention are preferably configured to be biocompatible and provide a service life suitable for episodic treatment evaluation of at least about 1-8 weeks, whether exposed to radiation, chemotherapy, heat or ionic electric fields (such as the treatment provided by a Thermotron® system) directed to the tumor. Additional description of suitable sensor units is found in U.S. Patent Nos. 6,402,689, 7,010,304, and 7,011,814, and in pending U.S. Patent Application Serial Nos. 10/551,366 and 10/779,907, the disclosures of which are incorporated by reference herein in their entireties. The sensor units are configured with internally mounted electronics that wirelessly communicate with an external reader. The sensor units can be configured as a miniaturized elongate (medical grade glass encapsulated or suitable aluminosilicate material) sensor body having a length of about 25 mm or less and a width of about 3mm or less implantable via a trocar. More particularly, in some embodiments, the sensor body may have a length of about 20 mm and a width of about 2.1 mm. The sensor body itself may be radio-opaque and/or may use radio-opaque coatings for visibility on CT or X-ray scans.

The sensor units can be configured as radiation sensors that can be used to detect, confirm, or verify irradiation doses delivered during photon irradiation treatment (cumulatively, typically in the range of between about 3000-6000 cGy, with each treatment optionally being increments of the total, such as 150-500 cGy). Thus, use of a radiation sensor during real time delivery can help control a more precise delivery dose of radiation to the tumor treatment site. Data regarding the distribution of dose within the tumor following irradiation and/or verification of a calculated or planned dose, may be particularly of interest as complex beam shaping, high dose conformal therapy, or beams at oblique angles may not consistently deliver the planned dose. In certain embodiments, the sensor units may be β radiation monitors used to monitor radioactively labeled compounds, drug uptake and/or utilization, blood flow in the tumor, sensitivity to specific drugs, drug distribution, labeled-glucose (or bio- constituent or metabolite thereof) or another analyte of interest in various locations or organs (as well as cell proliferation as discussed above).

Patients according to the present invention can be any animal subject, and are preferably mammalian subjects (e.g., humans, canines, felines, bovines, caprines, ovines, equines, rodents, porcines, and/or lagomorphs), and more preferably are human subjects.

The present invention is described herein with reference to flowchart illustrations and/or block diagrams of operations, methods and computer program products according to embodiments of the invention. It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, embedded processor or other programmable data processing apparatus to produce a machine, such that the instructions, which execute via the processor of the computer or other programmable data processing apparatus, create means for implementing the functions specified in the flowchart and/or block diagram block or blocks.

These computer program instructions may also be stored in a computer-readable memory that can direct a computer or other programmable data processing apparatus to function in a particular manner, such that the instructions stored in the computer-readable memory produce an article of manufacture including instruction means which implement the function specified in the flowchart and/or block diagram block or blocks.

The computer program instructions may also be loaded onto a computer or other programmable data processing apparatus to cause a series of operational steps to be performed on the computer or other programmable apparatus to produce a computer implemented process such that the instructions which execute on the computer or other programmable apparatus provide steps for implementing the functions specified in the flowchart and/or block diagram block or blocks.

As will be appreciated by one of skill in the art, the present invention may be embodied as a system, method, data or signal processing system, or computer program product. Accordingly, the present invention may take the form of an embodiment combining software and hardware aspects. Furthermore, the present invention may, in part, take the form of a computer program product on a computer-usable storage medium having computer-usable program code means embodied in the medium. Any suitable computer readable medium may be utilized including hard disks, CD-ROMs, optical storage devices, or magnetic storage devices.

The computer-usable or computer-readable medium may be, for example but not limited to, an electronic, magnetic, optical, electromagnetic, infrared, or semiconductor system, apparatus, device, or propagation medium. More specific examples (a non-exhaustive list) of the computer-readable medium would include the following: an electrical connection having one or more wires, a portable computer diskette, a random access memory (RAM), a read-only memory (ROM), an erasable programmable read-only memory (EPROM or Flash memory), an optical fiber, and a portable compact disc read-only memory (CD-ROM). Note that the computer-usable or computer-readable medium could even be paper or another suitable medium, upon which the program is printed, as the program can be electronically captured, via, for instance, optical scanning of the paper or other medium, then compiled, interpreted or otherwise processed in a suitable manner if necessary, and then stored in a computer memory.

Computer program code for carrying out operations of the present invention may be written in an object oriented programming language such as Java7, Smalltalk or C++. However, the computer program code for carrying out operations of the present invention may also be written in conventional procedural programming languages, such as the "C" programming language or even assembly language. The program code may execute entirely on the user's computer, partly on the user's computer, as a stand-alone software package, partly on the user' s computer and partly on a remote computer or entirely on the remote computer. In the latter scenario, the remote computer may be connected to the user' s computer through a local area network (LAN) or a wide area network (WAN), or the connection may be made to an external computer (for example, through the Internet using an Internet Service Provider).

The flowcharts and block diagrams used herein illustrate systems and/or operations that can be used to obtain and/or analyze localization data for telemetric (wirelessly operated) implantable sensor units. The analysis of the localization and a data can be carried out to provide real-time data on spatial location (i. e., movement) of a tumor during active delivery of a therapy such as, but not limited to, external beam radiation therapy. This spatial data can be interfaced with a delivery system to control the delivery of the therapy based on the spatial location of the tumor. For example, the spatial data can be used with a radiation (external beam) therapy system to thereby control, direct, guide, and/or gate (gate meaning direct the "on" or "off" of a radiation beam transmitted into the body) during an external beam radiation therapy session.

The flowcharts and block diagrams used herein also illustrate systems and/or operations that telemetrically obtain and/or analyze data associated with radiation measurements from the telemetric implantable sensor units. In this regard, each block in the flow charts or block diagrams represents a module, segment, or portion of code, which comprises one or more executable instructions for implementing the specified logical function(s). It should also be noted that in some alternative implementations, the functions noted in the blocks may occur out of the order noted in the figures. For example, two blocks shown in succession may be executed substantially concurrently or the blocks may sometimes be executed in the reverse order, depending upon the functionality involved.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and/or the present specification and will not be interpreted in an idealized or overly formal sense unless expressly so defined herein.

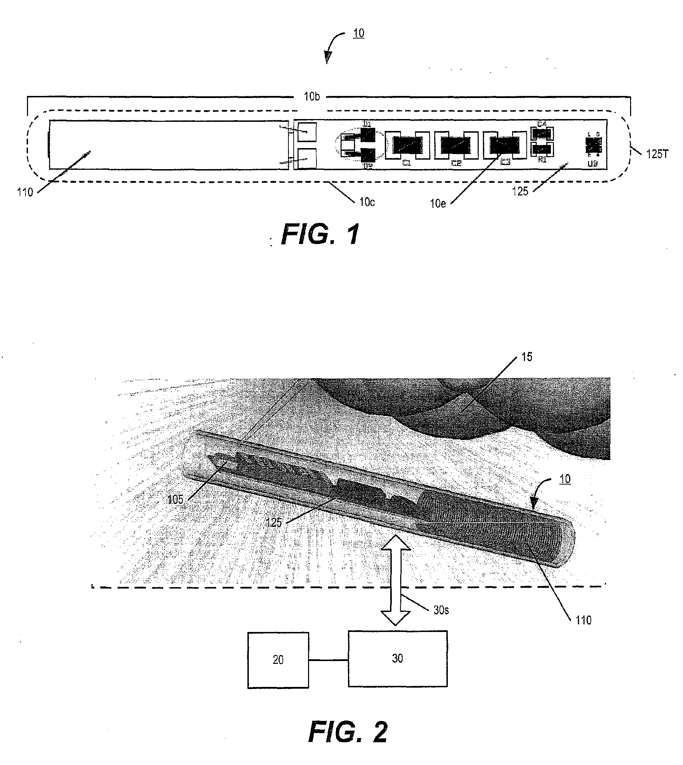

**Figures 1-12** illustrate devices, systems, methods and computer program products of embodiments of the present invention that provide implantable sensor units that can be implanted in the body to act as fiducial markers which are detectable using a desired imaging modality, such as, but not limited to, MRI, CT, MV, X-ray, RF, ultrasound, and/or other means that provide images of spatial location. As such, the sensor units can be a positional marker and can detect predetermined parameters *in vivo.* For example, the sensor units can be configured to detect changes of selected parameters associated with a delivered radiation dose. More particularly, the sensor units may be configured to guide and/or determine delivered external beam radiation dose(s) relative to the location of a target tumor treatment site, for example, to compensate for patient motion.

Turning now to the figures. **Figure 1** illustrates an implantable sensor unit **10.** The sensor unit **10** can be surgically implanted in an area of interest in a patient's body and may be telemetrically operated. More particularly, the sensor unit **10** includes electronics **10e** and antenna or transmitter coil **110** formed of a high-atomic weight element and configured to allow wireless communication to an external reader. In some embodiments, the sensor unit **10** includes one or more sensor elements configured to sense radiation and, as such, may also include a RADFET that operates with a threshold voltage "Vth" shift that is proportional to absorbed radiation dose. In certain embodiments, at least one, and typically a plurality of the implanted sensor units **10** can be configured to monitor fluorescence, radiation dose, and/or temperature. As such, the sensor **10** can include a RADFET, an optical detector and light source, and/or a digital temperature sensor as the sensor elements. For example, the temperature data and/or radiation dose data can be used to help administer hyperthermia/radiation combination therapies. The sensor unit 10 may also be inductively powered via the internal transmitter coil **110.**

As shown in **Figure 1****,** the sensor unit 10 is cylindrically shaped and sized for injection into the body of a patient. The electronics **10e** includes a PCB and/or IC chip **125,** such as an application-specific integrated circuit(ASIC), oriented to extend a small distance along a length of the body **10b** of the sensor unit **10.** The coil **110** also cylindrically extends adjacent to a portion of the PCB or IC **125.** In the embodiment shown, the PCB **125** is a substrate, such as a ceramic, flexible, or FR4 substrate, and the coil assembly (including the coil **110** and a core material **107)** is glued and/or otherwise secured to the PCB **125.** Of course, with the use of an IC configuration, this size may be further reduced. The sensor body **10b** can be configured to hold a single channel (*i.e.*, one sensor element for a PCB version having a width of about 1.5 mm) or multi-channel (multiple elements, with each channel layed side by side, and typically wider than the single channel version). For example, the sensory body **10b** may include an ASIC **125** including four measurement channels-1 temperature, 2 RADFET channels, and an input voltage measurement channel. The number of measurement channels may not affect the size of the sensor unit **10.** The tip **125T** of the sensor unit **10** can be configured with a rounded or pointed edge to help facilitate entry into the tumor tissue and/or into tissue adjacent to the tumor tissue.

As mentioned above, the sensor unit **10** includes a high-atomic weight member **110** within the sensor body **10b** that can be detected by an imaging modality. As used herein, a "high-atomic weight" member or element may refer to an element (and/or alloys/mixtures thereof) having an atomic weight of greater than about 180 atomic mass units (amu) and having a sufficient radio opacity to provide visual contrast when imaged using an imaging modality such as MV imaging. Examples of such high-atomic weight elements may include gold (Au) and platinum (Pt). As shown in **Figure 1****,** the high-atomic weight member **110** is provided as part of the transmitter coil. For example, the winding of the transmitter coil **110** may be formed of insulated gold wire. However, in other embodiments, the high-atomic weight member **110** may be provided as a different and/or separate component than the transmitter coil that is included in the housing of the sensor unit **10.**

The sensor unit **10** can be held in a hermetically sealed encapsulated housing **10c,** such as a glass capsule or other medically suitable material that is substantially impermeable. The sensor unit **10** electronics **10e** can, for example, include a microprocessor controller that controls data acquisition and reader/sensor unit communications that can be mounted on a ceramic substrate. The electronics **10e** can include custom chip designs with routings to semiconductor chips provided for data acquisition. For example, the electronics **10e** may include an ASIC that includes data acquisition and communications electronics on a single chip. The sensor unit **10** can include a bidirectional antenna. The sensor unit **10** can be configured with digital communication components (such as a digital signal processor) using a 12-16 bit data acquisition that can provide about a 1 mV or less resolution (or better) of the Vth measurement and may operate with a 16-bit CRC error checking capacity. For example, in some embodiments, the sensor unit **10** may include a 14-bit ADC with about 0.26 mV resolution. The electronics 10e can be potted in Class VI USP epoxy and hermetically sealed inside the capsule **10c.** The external surface or body of the capsule can be coated with a Parylene C material or other biocompatible coating. The sensor unit can be EO sterilized and adapted to be suitable for *chronic in vivo* implantation as described. The sensor body itself (or portions of the sensor body) may be radio-opaque for visual contrast in CT scans and port films and the like. Additional description of exemplary sensor unit housing configurations can be found in co-pending United States Patent Application Serial No. 10/353,857, the disclosure of which is incorporated by reference herein in its entirety.

**Figure 2** illustrates the sensor unit 10 of **Figure 1** implanted proximate a target site of interest, such as a tumor treatment site **15.** In some embodiments, the sensor unit **10** may be sized and configured to be injected to its position proximate the tumor treatment site **15.** In operation, the electronics **10e** and the transmitter coil **110** of the sensor unit **10** provide wireless communication with an external reader **30** using RF signals **30s** to relay data associated with at least one internal parameter of interest that is sensed via a sensor element **105.** The sensor unit **10** may also be inductively powered by the reader **30.** That is, the reader **30** may be configured to act as a transformer to couple and power the internally disposed sensors, as is well known to those of skill in the art. As such, *the in situ* sensor unit **10** may be self-contained, may have a sufficiently long service life in the body to provide clinically useful chronic information for episodic or chronic treatment decisions, and can be miniaturized without requiring catheters or externally connected wire leads into the sensors and out of the body.

For systems where multiple sensors **10** are used, the external reader **30** can be configured to serially poll each sensor at the same frequency, with each sensor having a unique RF identification data bit or bits allowing identification and individual interrogation. The multiple sensors may be placed adjacent or in tumors or proximate normal or non-targeted tissue or organs. Such placement can be selected to allow for external monitoring of doses received proximate sensitive and/or non-targeted sites, such as the thyroid, heart, or proximate the tumor or targeted treatment region.

The sensor unit **10** further includes at least one high-atomic weight member **110** that is configured to be detected by an imaging modality (not shown), such as MV, CT, X-ray, and/or other means that provide images of spatial location. For example, the high-atomic weight member may be gold (Au) and/or platinum (Pt). As shown in **Figure 2****,** the high-atomic weight member **110** is provided as a transmitter coil wound with insulated gold wire. However, the high-atomic weight member may be provided as a separate component from the coil in some embodiments, as will be discussed in greater detail below.

A spatial data processor **20** may be configured to determine the position and/or orientation of the sensor based on detection of the high-atomic weight member **110** via the imaging modality. In some embodiments, the external reader **30** and the spatial data processor **20** may be housed in the same primary housing unit and/or may share the same or portions of the same data or computer processing system. In other embodiments, the external reader **30** and the spatial data processor **20** may be separate units held in different housings. For example, the external reader **30** may be included in a radiation dose reader, a separate reader, and/or a combined reader, while the spatial data processor **20** may be included in the imaging modality.

For additional description of the sensor unit **10** and reader **30,** *see* U.S. Patent No. 6,402,689 and co-pending U.S. Patent Application Serial No. 10/127,207; the contents of these documents are hereby incorporated by reference as if recited in full herein. Suitable sensor units and telemetric readers may be provided by the DVS® system available from Sicel Technologies, Inc., located in Morrisville, N C. For example, in some embodiments, the sensor unit **10** may be a miniature DVS® sensor manufactured by Sicel Technologies, Inc. The sensor unit **10** may have dimensions of about 20 mm x 2.1 mm, and may be configured to pinpoint the tumor treatment site **15** during a patient's treatment cycle and also to measure the amount of radiation received at or proximate to the tumor treatment site **15.** Accordingly, sensor units according to some embodiments of the present invention may facilitate the visualization of tumor localization and provide actual dose information at the tumor location, which may be beneficial to accuracy in providing radiation therapy treatment.

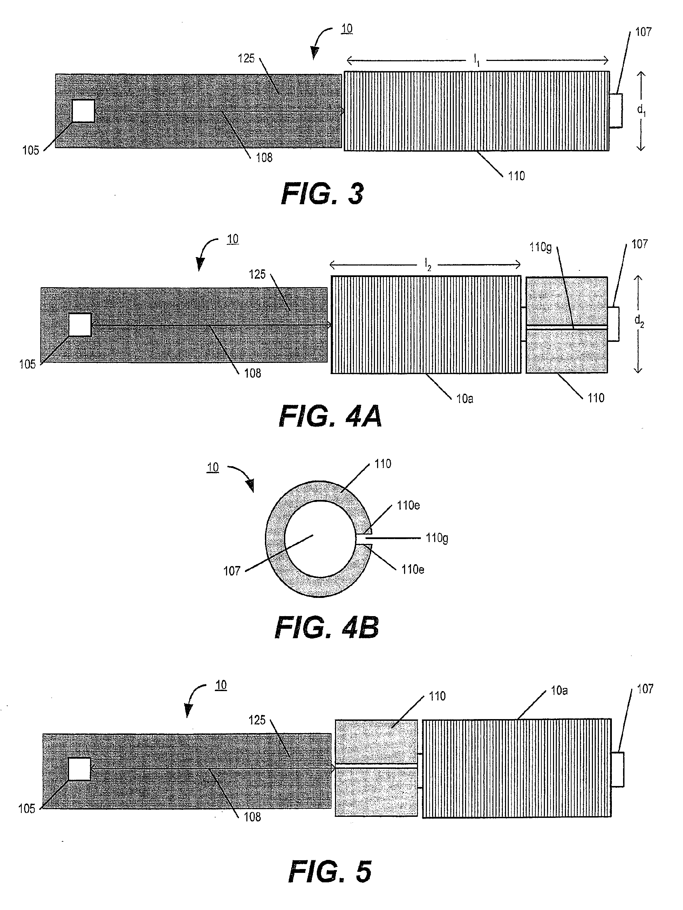

**Figure 3** is a partial top view illustrating the internal components of an example implantable sensor unit where the high-atomic weight member **110** is provided as a transmitter coil. The transmitter coil **110** is thus detectable as a fiducial marker in images generated using an imaging modality, such as CT, X-ray, and/or megavolt (MV) radiation therapy. More particularly, as shown in **Figure 3****,** the coil **110** is wound with insulated gold wire to surround a core material **107,** such as a ferrite core, and is glued or otherwise secured adjacent an end portion of the PCB **125.** In some embodiments, the core **107** may not overlap with the PCB **125.** Since the gold wire has a higher atomic weight than conventional copper wire, it is more radio opaque, and thus provides better contrast when imaged with high energy X-rays and/or other imaging modalities. By including the high-atomic weight member **110** in the transmitter coil, a location-trackable sensor unit may be fabricated according to some embodiments of the present invention without the use of additional components. However, since copper and/or other materials may be better conductors than gold, the read range (i. e., the distance between the external reader **30** of **Figure 2** and the implanted sensor unit **10)** may be slightly reduced with the gold coil design. For example, the read range for a sensor unit **10** including a gold coil may be about 11cm or less, as compared to a read range of about 12-14 cm or less for a sensor unit including a conventional copper coil.

Still referring to **Figure 3****,** at least one sensor element **105** is provided at an end portion of the PCB **125** opposite the coil **110.** For example, the sensor element **105** may be configured to detect beta, photon, and/or gamma radiation used in radiation therapies. In addition, the sensor element **105** may be configured to detect fluorescence at predetermined wavelengths of interest, for example, as exhibited by therapeutic antibodies used in cancer treatment. A minimum distance **108** may be maintained between the coil **110** and the sensor element **105** to inhibit and/or prevent secondary electron scattering off the coil **110,** which may affect calibration of the sensor element **105.** The calibration process may somewhat compensate for scattering; however, since scattering is a function of photon beam energy, the dose response energy dependency of the sensor element **105** may increase based on the proximity of the coil **110.** As such, the coil **110** may not be placed directly adjacent to the sensor element **105.** For example, the sensor element **105** and the coil **110** may be axially spaced apart by a distance of about 9 mm in some embodiments.

**Figure 4A** is a partial top view illustrating the internal components of an example implantable sensor unit including high-atomic weight members according to other embodiments of the present invention. As shown in **Figure 4A****,** the high-atomic weight member **110** of the sensor unit **10** is provided as a substantially cylindrical clip surrounding an end portion of the core **107** of the coil **10a.** For example, in some embodiments, the clip **110** may be formed of gold and/or other high-atomic weight material. Providing the high-atomic weight member **110** as a clip (rather than as the coil **110** illustrated in **Figure 3****)** may offer improved read range performance for use with an external reader (such as the reader **30** of **Figure 2****),** as the transmitter coil **10a** may be formed of copper and/or other elements having a higher conductivity than gold. The transmitter coil **10a** may also have a reduced length l₂ and an increased diameter d₂ (as compared to the length 1₁ and the diameter d₁ of the coil **110** of **Figure** 3) such that the clip **110** and the coil **10a** may occupy a substantially similar portion of the sensor unit **10** while maintaining a desired coupling strength for the coil **10a.** For example, where the sensor unit **10** is a Sicel Technologies DVS® sensor, the maximum diameter d₂ may be about 1.68mm, based on the size of the capsule **(10c** of **Figure 1****).** The clip **110** may be sized similarly to conventional gold markers to provide compatibility with existing software localization products and/or imaging modalities. Also, as discussed with respect to the embodiments of **Figure 3****,** at least one sensor element **105** is provided at the opposite end of the PCB **125.** As such, the distance **108** between the sensor element **105** and the clip **110** may be maximized, which may reduce the effects of secondary electron scattering on the sensor element **105.**

**Figure 4B** is a partial end view of the implantable sensor unit **10** of **Figure 4A****.** As shown in **Figure 4B****,** the clip **110** snugly resides against a portion of a substantially cylindrical antenna core **107.** The core **107** also extends at least partially through the coil **10a.** The clip **110** further includes an axially extending gap **110g** that separates long edges **110e** of the clip **110** from each other. The gap **110g** is sufficient to prevent an electrical connection between the long edges **110e** of the clip **110,** which may create a shorted turn around the antenna core **107.** Such a shorted turn may reduce the effective electromagnetic coupling between the coil **10a** and a reader antenna. As such, the gap **110g** may be filled with a non-conductive material. For example, in some embodiments, the gap **110g** may be filled with a gas, such as air. In addition, the gap **110g** may be filled with an insulator, such as rubber or plastic.

**Figure 5** is a partial top view illustrating the internal components of an example implantable sensor unit according to further embodiments of the present invention. As shown in **Figure 5****,** the high-atomic weight member **110** of the sensor unit **10** is provided as a clip surrounding a portion of the PCB **125** between the transmitter coil **10a** and the sensor element **105.** The coil **10a** may be wound with copper wire to surround an end portion of the PCB **125,** and the sensor element **105** may be provided at an end portion of the PCB **125** opposite the coil **10a.** The clip **110** and the sensor element **105** may be axially spaced apart by a distance **108** that is sufficient to reduce and/or prevent secondary electron scattering off the gold clip **110** from affecting calibration of the sensor element **105.** For example, the sensor element **105** and the clip **110** may be axially separated by a distance of about 9 mm in some embodiments.

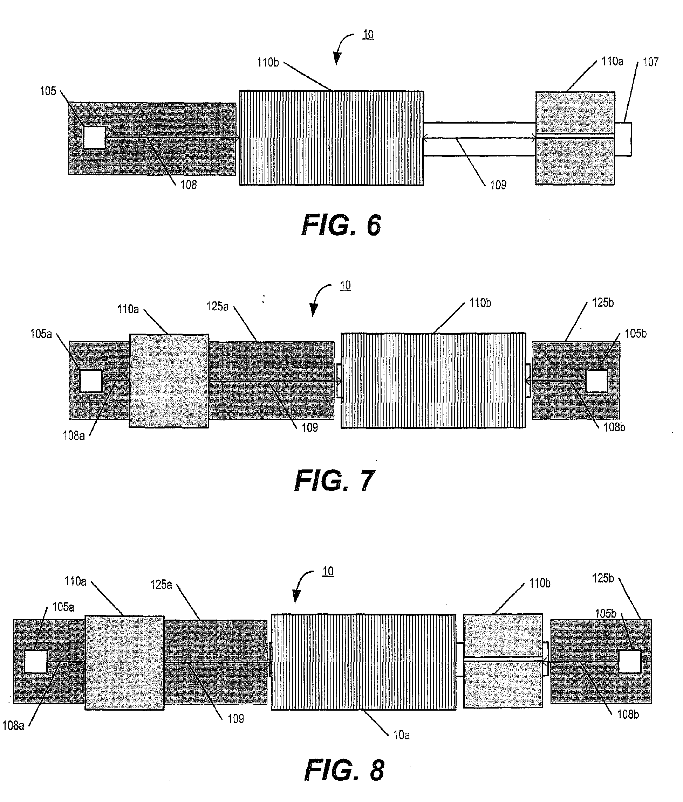

**Figures 6-8** are partial top views illustrating the internal components of example implantable sensor units including multiple high-atomic weight members according to still further embodiments of the present invention. More particularly, as shown in **Figure 6****,** the sensor unit **10** includes two high-atomic weight members, shown as clip **110a** and coil **110b,** axially spaced apart from one another along the core **107.** For example, one or both of the coil **110b** and the clip **110a** may be formed of gold and/or other high-atomic weight material. As such, the coil **110b** and the clip **110a** may both be detected as fiducial markers by an imaging modality. The detection of two axially-spaced apart points of the sensor unit **10** may be used to define a line representing a present orientation of the implanted sensor unit 10 inside the patient's body. For example, a spatial data processor, such as the spatial data processor **20** of **Figure 2****,** may be configured to define an orientation of the sensor body **10b** using positional data derived from detection of the coil **110b** and the clip **110a.** A sufficient axial spacing **109** may be provided between the coil **110b** and the clip **110a** for the spatial data processor to define the orientation of the sensor body. Also, the coil **110b** and a sensor element **105** at an end of the PCB **125** may be axially spaced apart by a distance **108** that is sufficient to reduce and/or prevent secondary electron scattering off the coil **110b** from affecting calibration of the sensor element **105.**

**Figure 7** illustrates a sensor unit **10** including two axially spaced apart high-atomic weight members, again shown as clip **110a** and coil **110b,** and two sensor elements 105a and **105b** at opposite ends of the sensor body. In particular embodiments, the sensor unit **10** includes a clip **110a** adjacent a sensor element **105a** at a first end of the sensor body, and a **coil 110b** adjacent a sensor element **105b** at a second end of the sensor body. The PCB includes first and second portions **125a** and **125b** secured to opposite ends of the coil **110b.** The clip **110a** surrounds at least a part of the first portion of the PCB **125a,** and does not include a gap between edges thereof. One or both of the coil **110b** and the clip **110a** may be formed of gold and/or other high-atomic weight material. As discussed above, the distance **108a** between the clip **110a** and the sensor element **105a** and the distance **108b** between the coil **110b** and the sensor element **105b** may be sufficient to reduce and/or prevent secondary electron scattering off the clip **110a** and the coil **110b** from affecting calibration of the sensor elements **105a** and **105b**, respectively.

Still referring to **Figure 7****,** the sensor elements **105a** and **105b** may respectively provide signals to an external reader (such as the reader **30** of **Figure 2****).** For example, the signals may include data corresponding to a radiation dose administered proximate the location of the sensor elements **105a** and **105b**. An axial spacing **109** may be also provided between the coil **110b** and the clip **110a** that is sufficient for a spatial data processor (such as the spatial data processor **20** of **Figure 2****)** to define the orientation of the sensor body. As such, based on the signals from the sensor elements **105a** and **105b** and the orientation of the sensor body, a radiation dose gradient measurement may be defined. The radiation dose gradient measurement may indicate the relative amounts of radiation being delivered proximate to the tumor treatment site based on two or more dose measurements.

**Figure 8** illustrates a sensor unit **10** including two high-atomic weight members, shown as two clips **110a** and **110b,** adjacent two sensor elements **105a** and **105b** at opposite ends of the sensor body. A copper transmitter coil **10a** is provided between the clips **110a** and **110b**. The signals from the sensor elements **105a** and **105b** and the orientation of the sensor body as determined from detection of the two axially-spaced apart clips **110a** and **110b** may be used to define a radiation dose gradient measurement in a manner similar to that described above with reference to **Figure 7****.**

Although illustrated above in **Figures 3-8** with reference to specific sensor configurations, it is to be understood that embodiments of the present invention may include any configuration having one or more high-atomic weight members in an implantable sensor unit that is configured to sense at least one internal parameter of interest.

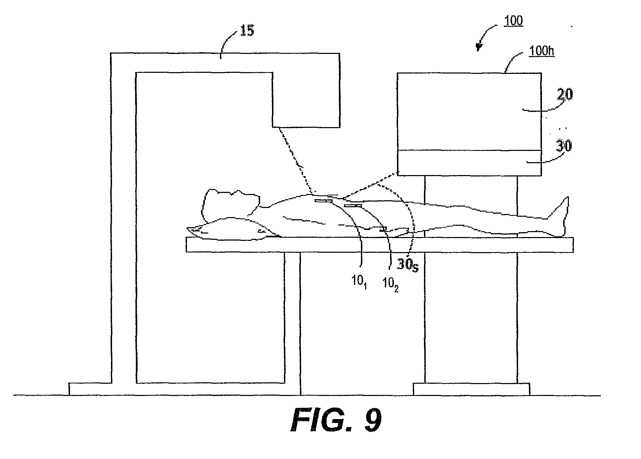

**Figure 9** illustrates a medical system **100** for obtaining intra-body data for least one sensed internal parameter as well as positional data that may be used as a therapy guide system according to some embodiments of the present invention. As shown in **Figure 9****,** in certain embodiments, the patient may be positioned in an imaging system **15.** In certain particular embodiments, the imaging system **15** may be a computed tomography (CT) system. Suitable radiation treatment systems for use with the medical system **100** include, but are not limited to, three-dimensional conformal external beam radiation, intensity modulated radiation therapy (IMRT), a "gamma knife" that employs a highly focused gamma ray radiation obtained from crossing or collimating several radiation beams, stereotactic radiosurgery and brachytherapy systems.

Referring to **Figure 9****,** the system **100** includes a spatial data processor **20,** one or more implanted telemetric sensor units (shown as two units **10₁, 10₂**), and an external reader **30** configured to receive wireless transmissions corresponding to data for at least one sensed internal parameter from the implanted sensor units **10.** The sensor units **10₁** and **10₂** may be similar and configuration and/or function to one or more of the sensor units **10** of **Figures 1-8****.** As such, the sensor units **10₁** and **10₂** each include at least one high-atomic weight member configured to be detected by the imaging system **15.** The system **100** can also include a primary housing **100h.** The housing **100h** may include user input controls and a display or other communication means as desired (not shown).

As illustrated in **Figure 9****,** the reader **30** inductively powers one or more of the implanted sensor units **10.** As such, the sensor units **10** wirelessly transmit data corresponding to at least one sensed internal parameter to the reader **30.** For example, the sensor units **10** may wirelessly transmit data associated with the dose and/or processing of a therapy administered to a tumor treatment site proximate to the implanted sensor units **10.** In addition, the high-atomic weight members included in the sensor units **10** are detectable as fiduicial markers by the imaging system **15.** As such, the spatial data processor **20** may determine positional and/or orientation data for the sensor units **10** using spatial data derived from detection of the sensor units **10** by the imaging system **15.** For example, the positional data and the dosing data for the sensors **10** can be used with a radiation therapy system to control the delivery of an external radiation beam into the body during an external beam radiation therapy session.

In certain embodiments, the system **100** can be configured to individually selectively (serially) poll, address, and/or interrogate a selected implanted sensor unit **10.** The sensor units **10** can be configured to operate or wirelessly communicate with the reader **30** at the same frequency. The read range between the external reader **30** and the implanted sensor units **10** may be between about 5cm-15cm, and is typically less than about 12 cm, depending on the particular configuration of the sensor units **10.** For example, the read range for a sensor unit **10** including a gold coil may be about 11 cm or less, as compared to a read range of about 12-14 cm or less for a sensor unit including a conventional copper coil. Also, the sensor units **10** may be implanted at least 3 cm deep. To control and/or identify which of the sensor units **10** are in active communication mode, a single or multi-bit identifier can be communicated to the reader **30** by the sensor units **10** in the data stream. For example, in some embodiments, each sensor unit **10** includes a unique 32-bit identifier, and the reader **30** addresses each sensor unit **10** using this identifier.

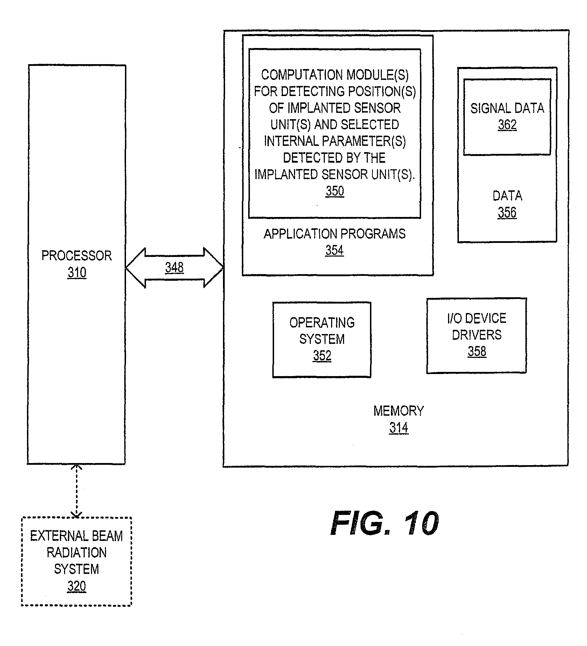

**Figure 10** is a block diagram of exemplary embodiments of data processing systems that include a computation module **350** in accordance with embodiments of the present invention. The processor **310** communicates with the memory **314** via an address/data bus **348.** The processor **310** can be any commercially available or custom microprocessor. The memory **314** is representative of the overall hierarchy of memory devices containing the software and data used to implement the functionality of the data processing system **305.** The memory **314** can include, but is not limited to, the following types of devices: cache, ROM, PROM, EPROM, EEPROM, flash memory, SRAM, and DRAM.

As shown in **Figure 10****,** the memory **314** may include several categories of software and data used in the data processing system **305:** the operating system **352;** the application programs 354; the input/output (I/O) device drivers **358;** a computation module **350;** and the data **356.** The data **356** may include signal data **362** which may be obtained directly from the implanted sensor unit(s). The computation module **350** includes computer program code that determines a spatial location of at least one implanted sensor unit based on detection of a high-atomic weight member therein by an imaging modality, and evaluates at least one internal selected parameter based on the signal data **362** provided by the at least one implanted sensor unit.

In certain embodiments, the selected internal parameter is radiation dose detected by the at least one implanted sensor unit. For example, where the sensor unit includes two axially spaced apart high-atomic weight members **110a** and **110b** (for instance, as illustrated in **Figure 7****),** the computation module **350** may determine an orientation of the sensor unit based on a line defined by the two detected high-atomic weight elements. Moreover, using the detected radiation dose from two sensor elements in the implanted sensor unit, the computation module **350** may determine a radiation dose gradient measurement based on the orientation of the sensor unit relative to the tumor treatment site. Accordingly, the computation module **350** can be used during delivery of focused therapies, such as radiation therapies, and can include real-time spatial data feedback that can identify whether the target tumor is moving and dynamically control the intensity and/or direction of delivery of the therapies and/or dynamically adjust patient location during delivery of the therapies based on movement of the tumor to correctly align the patient and the beam. More particularly, the processor **310** may communicate with an external beam radiation therapy delivery system **320** to adjust delivery of the radiation therapy based on the spatial location(s) determined by the computation module **350.**

As will be appreciated by those of skill in the art, the operating system **352** may be any operating system suitable for use with a data processing system, such as OS/2, AIX or OS/390 from International Business Machines Corporation, Armonk, NY, WindowsCE, WindowsNT, Windows95, Windows98, Windows2000, WindowsXP or Windows XT from Microsoft Corporation, Redmond, WA, PalmOS from Palm, Inc., MacOS from Apple Computer, UNIX, FreeBSD, or Linux, proprietary operating systems or dedicated operating systems, for example, for embedded data processing systems.

The I/O device drivers **358** typically include software routines accessed through the operating system **352** by the application programs **354** to communicate with devices such as I/O data port(s), data storage **356** and certain memory **314** components and/or the image acquisition system **320.** The application programs **354** are illustrative of the programs that implement the various features of the data processing system **305** and preferably include at least one application that supports operations according to embodiments of the present invention. Finally, the data **356** represents the static and dynamic data used by the application programs **354,** the operating system **352,** the I/O device drivers **358,** and other software programs that may reside in the memory **314.**

The I/O data port(s) can be used to transfer information between the data processing system and another computer system or a network (e.g., the Internet) or to other devices controlled by the processor. These components may be conventional components such as those used in many conventional data processing systems, which may be configured in accordance with the present invention to operate as described herein.

While the present invention is illustrated, for example, with reference to the computation module **350** being an application program in **Figure 10****,** as will be appreciated by those of skill in the art, other configurations may also be utilized while still benefiting from the teachings of the present invention. For example, the module **350** may also be incorporated into the operating system **352,** the I/O device drivers **358** or other such logical division of the data processing system **305.** Thus, the present invention should not be construed as limited to the configuration of **Figure 10****,** but is intended to encompass any configuration capable of carrying out the operations described herein.

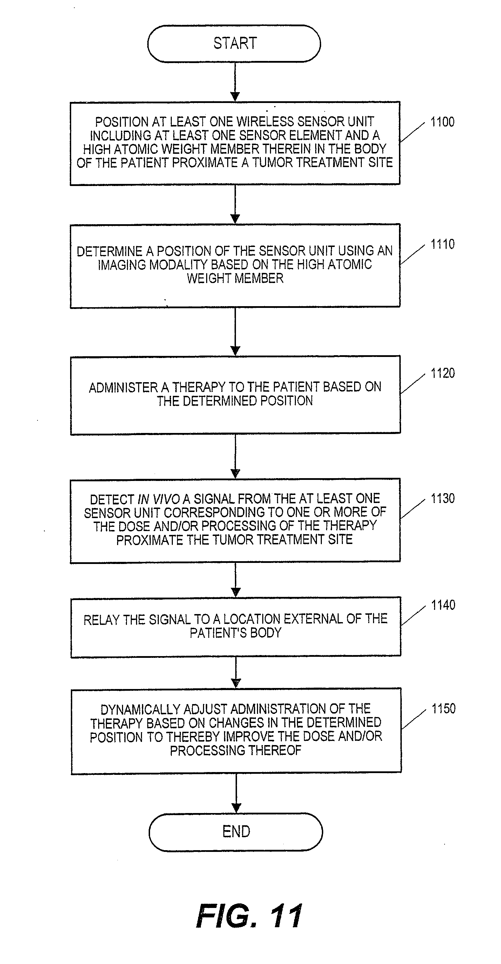

**Figure 11** illustrates operations for treating a patient for cancer that can be carried out according to embodiments of the present invention. As shown in **Figure 11****,** at least one wireless sensor unit is positioned in the body of a patient proximate a tumor treatment site (block **1100).** The wireless sensor unit includes one or more sensor elements configured to provide data corresponding to at least one sensed internal parameter. The wireless sensor unit also includes at least one member or component formed of a high-atomic weight element, such as gold and/or platinum. For example, the high-atomic weight member may be provided as a clip within the sensor unit and/or as part of the transmitter coil of the sensor unit that is used to wirelessly transmit the data corresponding to the sensed internal parameter(s). Accordingly, a position of the sensor unit is detected using an imaging modality based on the presence of the high-atomic weight member (block **1110).** For example, the high-atomic weight member may be detected as a fiducial marker in images generated using computed tomography (CT), megavolt (MV) radiation therapy, and/or other imaging modalities. A therapy is administered to the patient based on the detected position of the high-atomic weight member (block **1120).** For example, the therapy may be radiation delivered by an external beam radiation therapy system. The intensity and/or direction of delivery of the therapy may also be dynamically adjusted based on the detected position of the sensor unit (which may indicate movement of the tumor) to correctly align the patient and the beam.

A signal corresponding to the dose and/or processing of the administered therapy proximate to the tumor treatment site is detected *in vivo* from the at least one sensor unit (block **1130).** For example, as discussed above, the sensor unit may include first and second to sensor elements in opposing ends thereof and may be configured to transmit a signal indicating the dose of radiation delivered to each sensor element. The signal(s) from the sensor unit(s) are relayed to a location external of the patient's body (block **1140).** For example, the signals may be relayed to an external reader, such as the reader **30** of **Figure 2****,** which may be part of a cancer therapy system, such as the system **100** of **Figure 9****.** Administration of the therapy may also be dynamically adjusted responsive to changes in the determined position of the sensor unit to thereby improve the dose and/or processing of the therapy (block **1150).** The system **100** may also be configured to define a radiation dose gradient measurement based on the signals from the first and second sensor elements and the detected position of the sensor unit.

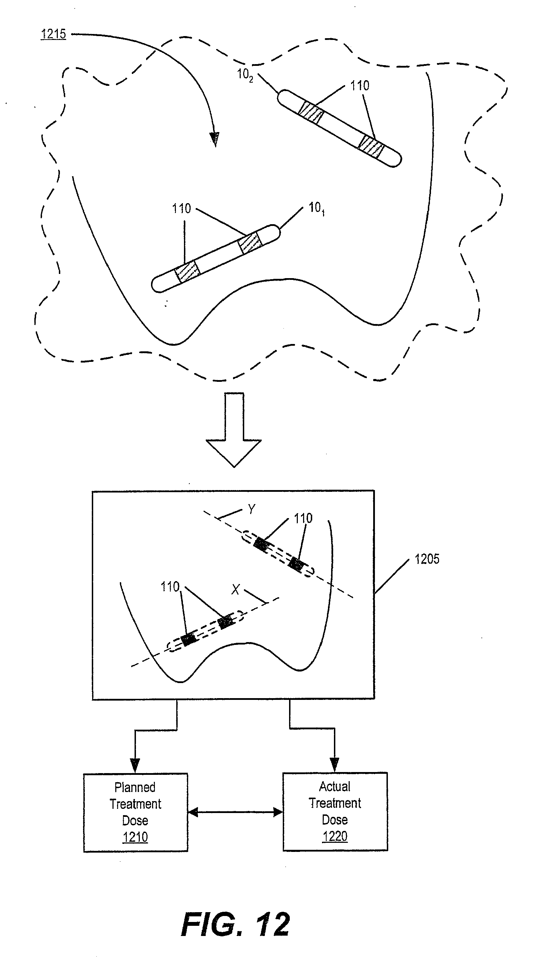

**Figure 12** illustrates an imaging display that provides real-time gradient dose radiation data relative to the spatial location of an internal tumor treatment site according to some embodiments of the present invention. As shown in **Figure 12****,** two sensor units **10₁** and **10₂** are implanted proximate a tumor treatment site **1215** in the body of a patient. Each of the sensor units **10₁** and **10₂** includes a pair of axially spaced apart high-atomic weight members **110** having a sufficient opacity to provide visual contrast when imaged using an imaging modality. Accordingly, the high-atomic weight members **110** are visible on an imaging display **1205** associated with the imaging modality. The orientations of each of the sensor units **10₁** and **10₂** may be determined from the lines *X* and *Y* defined by the pairs of high-atomic weight members **110** in the sensors **10₁** and **10₂,** respectively. As such, when a radiation therapy is administered to the patient, radiation dose gradient measurements may be determined based on the orientations of the sensors **10₁** and **10₂** relative to the tumor treatment site **1215** and detected radiation doses from sensor elements in the implanted sensor units 10₁ and 10₂, as discussed above. An actual treatment dose **1220** provided to the tumor treatment site may thereby be determined based on the radiation dose gradient measurements, and may be compared to a planned treatment dose **1210**. As such, if the actual treatment dose **1220** varies from the planned treatment dose **1210**, patient setup and/or treatment planning may be altered and/or reevaluated. Also, as discussed above, delivery of a radiation therapy may be dynamically adjusted based on the orientations of the sensor units **10₁** and **10₂** provided by the imaging display 1205 as detected during radiation therapy to align the patient and the radiation beam to provide the planned treatment dose **1210** to the tumor treatment site **1215.**

The foregoing is illustrative of the present invention and is not to be construed as limiting thereof. Although a few exemplary embodiments of this invention have been described, those skilled in the art will readily appreciate that many modifications are possible in the exemplary embodiments without materially departing from the novel teachings and advantages of this invention. Accordingly, all such modifications are intended to be included within the scope of this invention as defined in the claims. In the claims, means-plus-function clauses are intended to cover the structures described herein as performing the recited function and not only structural equivalents but also equivalent structures. Therefore, it is to be understood that the foregoing is illustrative of the present invention and is not to be construed as limited to the specific embodiments disclosed, and that modifications to the disclosed embodiments, as well as other embodiments, are intended to be included within the scope of the appended claims. The invention is defined by the following claims, with equivalents of the claims to be included therein.

## Claims

1. An implantable biocompatible sensor unit, comprising:
a sensor body comprising a biocompatible material and having at least one sensor element configured for *in vivo* placement proximate a tumor treatment site and configured to provide data corresponding to treatment thereof;
a transmitter coil and associated electronic components within the sensor body configured for wireless transmittal of the data to a spatially remote receiver; and
a high-atomic weight member within the sensor body configured to be detectable by an imaging modality,
wherein the sensor unit is configured to be inductively powered to wirelessly transmit the data to the remote receiver while remaining implanted proximate the tumor treatment site.

2. An implantable biocompatible sensor unit according to Claim 1, further comprising:
a second high-atomic weight member within the sensor body configured to be detectable by the imaging modality and axially spaced apart from the first high-atomic weight member.

3. An implantable biocompatible sensor unit according to Claim 2, wherein the at least one sensor element comprises first and second axially spaced apart sensor elements configured to respectively provide radiation data to the remote receiver to define a radiation dose gradient measurement proximate the tumor treatment site based on the radiation data and an orientation of the sensor body determined from detection of the first and second high-atomic weight members.

4. An implantable biocompatible sensor unit according to Claim 1, wherein at least a portion of the transmitter coil comprises the high-atomic weight member.

5. An implantable biocompatible sensor unit according to Claim 1, wherein the high-atomic weight member comprises a clip snugly residing against a substantially cylindrical antenna core extending within at least a portion of the sensor body and at least partially through the transmitter coil.

6. An implantable biocompatible sensor unit according to Claim 5, wherein the sensor body comprises a substantially cylindrically shaped body having opposing first and second ends, wherein the clip is substantially cylindrical and resides within the first end of the sensor body, and, preferably, wherein the clip includes an axially extending gap configured to separate long edges of the clip.

7. An implantable biocompatible sensor unit according to Claim 5, wherein the transmitter coil comprises insulated gold wire, and, preferably, wherein the transmitter coil is located at a first end of the sensor body and wherein the clip is located at a second end of the sensor body axially spaced apart from the first end.

8. An implantable biocompatible sensor unit according to Claim 1, wherein the high-atomic weight member comprises at least one of gold and/or platinum, and/or wherein the sensor element is configured to detect beta, photon, and/or gamma radiation.

9. An implantable biocompatible sensor unit according to Claim 1, wherein the sensor body is configured to periodically output the data over time to provide substantially real-time data of the internal condition of a tumor or tissue proximate thereto.

10. A medical system for patients undergoing treatment for cancer, the system comprising:
at least one wireless implanted sensor unit configured for in vivo placement proximate a tumor treatment site, the at least one sensor unit being configured to provide data including at least one sensed internal parameter, the at least one sensor unit including a high-atomic weight member held in a biocompatible housing and
configured to be visualized as a fiducial marker by an imaging modality, wherein the at least one sensor unit is configured to be inductively powered to wirelessly transmit the data; and
an external reader configured to inductively power the at least one sensor unit and configured to receive the data from the at least one sensor unit,
wherein the system is configured to dynamically monitor selected in vivo parameters associated with one or more of dose and/or processing of a therapy administered to the tumor treatment site based on the data from the at least one sensor unit.

11. A system according to Claim 10, wherein the high-atomic weight member is detectable in images generated using computed tomography (CT), megavolt (MV) radiation therapy, and/or other imaging modalities, and/or wherein the system is configured to dynamically adjust administration of the therapy to thereby improve localized delivery of radiation dose responsive to detection of the high-atomic weight member in the sensor unit.

12. A system according to Claim 10, wherein the at least one wireless implanted sensor unit further comprises a second high-atomic weight member held in the biocompatible housing axially spaced apart from the first high-atomic weight member and configured to be detected by the imaging modality, or wherein the high atomic weight member comprises a clip snugly residing against a substantially cylindrical antenna core extending within at least a portion of the server unit.

13. A system according to Claim 12, wherein the system is configured to define an orientation of the sensor body using spatial data derived from detection of the first and second high-atomic weight members by the imaging modality, and, preferably, wherein the at least one sensor unit comprises first and second axially spaced apart sensor elements configured to respectively provide radiation data to the external reader to define a radiation dose gradient measurement based on the orientation of the sensor unit.

14. A method for treating a patient for cancer, the method comprising:
positioning at least one wireless sensor unit including at least one sensor element and a high-atomic weight member therein in the body of the patient proximate a tumor treatment site;
determining a position of the sensor unit proximate to the tumor treatment site using an imaging modality based on the high-atomic weight member;
administering a radiation therapy to the patient based on the determined position;
detecting *in vivo* a signal from the at least one sensor unit corresponding to the dose of the administered therapy proximate the tumor treatment site;
relaying the signal to a location external of the patient's body; and
dynamically adjusting administration of the therapy to thereby improve the localized delivery of radiation dose based on changes in the determined position.

15. A method according to Claim 14, wherein the sensor unit includes a second high-atomic weight member therein axially spaced apart from the first high-atomic weight member, wherein the at least one sensor element comprises first and second axially spaced apart sensor elements, and further comprising:
determining an orientation of the sensor body using spatial data derived from the determined position of the first and second high-atomic weight members by the imaging modality; and
defining a radiation dose gradient measurement based on the signal from the sensor unit and the determined orientation of the sensor body.
